# EUROPEAN PATENT APPLICATION

(11) **EP 4 733 010 A1**
(43) Date of publication of application: **29.04.2026**
(21) Application number: 24208803.7
(22) Date of filing: 24.10.2024
(51) Int. Cl.: B25J 5/00, A61B 34/30, B25J 11/00, B25J 19/00

(54) **AUTONOMOUS MOBILE PLATFORM FOR MEDICAL USE**

(71) Applicant: Ronna Medical d.o.o., 10000 Zagreb (HR)
(72) Inventor: Jerbic, Bojan, 10000 Zagreb (HR); Klaric, Igor, 10000 Zagreb (HR); Zupancic, Ivan, 10000 Zagreb (HR); Sekoranja, Bojan, 10000 Zagreb (HR)
(74) Representative: Bihar, Zeljko

(57) **Abstract**

An autonomous mobile platform (100) is disclosed. It comprises a loading platform (10), an upper chassis (20), a lower chassis (30), two centrally positioned independently powered drive wheels (51, 61) with suspension means, a set of omni-wheels (70), a rotating plate (80) and a set of lifting means (40). The synchronous engagement of all lifting means (40) in one direction lifts the connected chassis (20, 30) towards the loading platform (10) and leaves the autonomous mobile platform (100) anchored on their anchoring legs (15) on the floor. The opposite action brings the platform wheels to the floor. The propelling and steering of the platform (100) are performed by the coordinated actions of the rotating plate (80), together with a separate independent rotation of the drive wheels (51, 61), and free rotation of the omni-wheels (70) on the floor. The platform is designed for carrying surgical equipment, especially surgical robots.

## Description

### Technical Field

The present disclosure relates to the field of medical robots intended to assist a practitioner during medical treatment. The disclosure relates, in particular, to autonomous mobile platforms capable of moving in any direction and equipped with a floor anchoring mechanism with advanced safety features.

### Technical Problem

Many medical robots are used nowadays to assist a practitioner during medical treatment. Most comprise an autonomous or man-propelled platform with one or more robotic arms attached to the said platform for performing the desired task, and a computer vision system for precisely locating the tools and the said platform to the patient and the practitioner, e.g., the surgeon.

The present disclosure solves a set of technical problems recognized in the practice, i.e., while using the autonomous mobile platform.

The most important technical problem solved with the present disclosure is how to anchor the mobile platform safely and reliably to the floor. Reliably, as used herein, has a meaning to anchor the platform to the floor without compromising the platform position, and some of the prior art platforms perform this task equally well. However, safety has a different interpretation in a what-if scenario. What if the platform once anchored to the floor, for unknown reasons or algorithm errors, engages the moving mechanism with full power? Will this action have any serious implications for the movable platform? How does this influence, for instance, ongoing brain surgery or similar very precisely executed medical treatments? What is the risk for the patient and the medical staff? The present disclosure eliminates such a risk with carefully designed anchoring means that retracts the platform's driving wheels and auxiliary stabilizing omni-wheels from the floor in the interior of the mobile platform to enable the floor anchoring process. It is worth noting that the mobile platform rests on the legs that are not part of the lifting mechanism, i.e., attached to the hydraulic and/or electric jack - which is common in the art and is a potential safety problem.

Furthermore, the disclosed movable platform is capable of executing an instant move in any desirable direction from the start. This is achieved with the use of two independent drives, i.e., the first and the second drives, both attached to the centrally positioned rotating platform nested in the center of the movable platform. For stability reasons, the set of omni-wheels is distributed over the platform. Attention is also paid to the suspension means used in the platform's drives, and those used in an auxiliary set of omni-wheels.

Keeping in mind the average weight of the disclosed platforms in the art, equipped with the system of robotics arms, end effectors, and computer vision systems, renders the manipulation of the platform complex. Namely, anchoring and de-anchoring can be problematic for platforms with 250-700 kg. Therefore, mechanical help - formed as an energy-storing mechanism - accompanied by lifting means should be employed to facilitate the anchoring and de-anchoring from the floor, as disclosed herein.

### State of the Art

Almost all surgical robots are somehow movable, i.e., attached to the propelling or movable platforms. Part of them is equipped with an anchoring system that immobilizes the platform over the floor, the two are mentioned below.

The European patent EP2157931B1 for MULTI-APPLICATION ROBOTISED PLATFORM FOR NEUROSURGERY AND RESETTING METHOD, filed in the name of MEDTECH S.A., FR, relates to a multi-application robotized platform for neurosurgery. The preferred anchoring system disclosed in this reference consists of the hydraulic circuit used to immobilize the platform over the floor. At least three legs are designed to bring the platform with the wheels up - once the legs are stretched out. In one variant, the electric jack is used instead of hydraulic jacks. The problem with this solution is that the whole platform rests on hydraulic and/or electric jacks which are capable of causing a mechanical failure, especially if hydraulics is used.

The European patent EP3856063B1 for MEDICAL ROBOT COMPRISING AUTOMATIC POSITIONING MEANS, filed in the name of QUANTUM SURGICAL, FR discloses a movable medical platform with the anchoring means. From the accepted claims, it is evident that the medical platform has retractable feet intended to be lowered in order to immobilize the mobile base of the medical robot. Each foot has a sensor that makes it possible to measure a weight supported by said foot and verify a stability criterion for each foot, by comparing the measurement of the weight supported by said foot against a predetermined threshold. A deeper description inspection reveals that the retractable feet are associated with hydraulic or electrical cylinders for lowering them into contact with the floor, to support all or a part of the weight of the medical robot.

Both above-described solutions are not immune to the potential failure of a hydraulic or electric jack, where the supporting legs are part of the lifting mechanism, which is not the case in the present disclosure. Namely, the resting position in the present disclosure is obtained in a manner that the driving mechanism is entirely retracted within the mobile platform, and the legs naturally support the chassis with the instrumentation, robotic arms, etc., as if all the said are lying on the ordinary table with an arbitrary number of supporting legs.

The propulsion system is also important for any medical platform. One of the requirements for the propulsion system is a high degree of maneuverability. The ideal movable medical platform should approach anyhow to the patient's table. In each instant of the time, it should be able to move in any desirable direction. A few examples of such a platform are given in the examples below.

US patent US 5,323,867 for ROBOT TRANSPORT PLATFORM WITH MULTIDIRECTIONAL WHEELS, filed in the name of E. J. Allard et al., US, teaches about a robot transport platform adapted for locomotion, having a base with three wheels on each side. Of the three wheels on each side, the wheels near the fore-end and the aft end are omnidirectional wheels. The intermediate wheel in between the omnidirectional wheels is a conventional wheel. The omnidirectional wheels have staggered rows of spherical rollers rotatably mounted to the circumference of the wheel's hub. More precisely, the rollers are mounted to annular shafts that circumscribe the circumference of the hub, and are supported in an overlying position over the hub by radially extending spokes. Torque is provided by two electric motors independently operating the wheels on each side. Torque is transferred to the wheels via a gearbox, a chain, and cogged drive belts.

JP patent application JP2014024434A for the invention CARRIER, granted as the JP patent JP5762367B2, and filed in the name of Toyota Motor East Japan Inc., JP. It teaches about the vehicle with a similar cinematic ability as the solution disclosed herein; with centrally positioned two drives and omni-wheels disposed on the corners. Each wheel has its suspension means, and the advantages are described in the description.

PCT patent application WO0246031A1 for REMOTE-OPERATED MULTIDIRECTIONAL TRANSPORT VEHICLE, filed in the name of E. J. Allard. The platform with three pairs of omni-wheels is disclosed, where the central pair of omni-wheels is situated perpendicularly to the other two pairs.

PCT patent Application WO2019183580A1 for ACCELERATION CONTROLS FOR MOBILE DRIVE UNIT, filed in the name of Amazon Technologies Inc., US. It teaches about the mobile unit with a central propulsion system and two pairs of caster wheels disposed front and rear over the chassis.

European patent EP3048009B1 for INVENTORY SYSTEM WITH MOBILE DRIVE UNIT AND INVENTORY HOLDER, filed in the name of Amazon Technologies Inc., US. The mobile base, which is designed to transport the inventory holder has the propulsion similar to those disclosed herein, with the centrally disposed driving units.

It is worth noting that none of the above-cited solutions uses a rotating plate for changing the direction of the driving wheels. Such a solution greatly improves the maneuverability of the disclosed mobile platform, as evident for the person skilled in the art.

### Summary of the Invention

The present invention relates to an autonomous mobile platform for medical use, capable of moving in any direction. It comprises a loading platform, a chassis, a cover, two drives, a set of omni-wheels, a rotating platform, and a set of lifting means.

Each drive that propels the platform has a driving wheel powered by an electromotor and is attached to the rotating platform by a suspension means. The rotating platform is nested within the chassis and rotated by the plate drive.

Each lifting means is fixed to the chassis and comprises an electromotor that powers a spindle connected to the loading platform and enables lifting and lowering of the chassis towards the loading platform.

Each omni-wheel is fixed with the chassis via wheel support and enclosed in a wheel casing. The cover is situated below the chassis and their distance is fixed. The loading platform, situated above the chassis, is stabilized within the said chassis via a set of guides and compatible guide rods. This allows the motion of the chassis toward the loading platform in a way to keeps the said platforms parallel. The loading platform is equipped with at least three anchoring legs of the same height that are longer than the distance between the cover and the chassis.

The core of the invention is in the following features. The synchronous engagement of the lifting means in one direction lifts the chassis towards the loading platform and leaves the autonomous mobile platform anchored on its anchoring legs on the floor, while the driving wheels and all omni-wheels are without any contact with the floor. The synchronous engagement of the lifting means in the opposite direction moves the chassis from the loading platform and leaves the autonomous mobile platform to stand on the driving wheels and all omni-wheels with all anchoring legs lifted from the floor.

In the preferred embodiment, each lifting means is equipped with a lifting spring situated over the spindle to assist with a load when the chassis is moved, with the lifting means, towards the loading platform, resulting in the mobile platform on its wheels. Also, due to high loads lifting propulsion is supplemented with lifting springs which store energy and enable the use of less powerful motors.

In the preferred embodiment, the propelling and steering of the platform are performed by the coordinated actions of the rotating plate and separate independent rotation of the left drive and the right drive, while the driving wheels and omni-wheels are on the floor. In one variant, the left drive and the right drive are equipped with suspension means formed from the suspension springs pivotally connected to the suspension levers holding the corresponding drive shafts suspended to the rotating plate.

In another embodiment, the omni-wheels are made of elastomers that act as a suspension means for the said omni-wheels. In yet another embodiment, the number of the omni-wheels is four and the number of the anchoring legs is three.

The autonomous mobile platform is intended to be used for carrying surgical equipment, especially surgical robots.

### Description of Figures

The set of figures that describe the disclosure is enumerated with the letter "A" when the mobile platform is in a position that allows propelling, and "B" when the mobile platform is anchored to the floor.
Figures 1A and 1B represent the perspective view of the mobile platform, where all essential parts are visible.
Figures 2A and 2B represent a cross-section made across the platform, substantially in the plane perpendicular to the loading platform and across to the diving wheels axles.
Figures 3A and 3B represent a side view of the mobile platform, and only on those figures, the floor is depicted as a straight line. The drive suspension sag is visible when comparing these two pictures.
Figures 4A and 4B represent a cross-section made across the platform, substantially in the plane perpendicular to the loading platform and across the first drive suspension means and a front omni-wheel.
Figures 5A and 5B represent a cross-section made across the platform, substantially in the plane perpendicular to the loading platform and across the lifting means and a back omni-wheel.
Figure 6 shows a mobile platform bottom with the distribution of omni-wheels, driving wheels with the corresponding suspensions, and anchoring legs in the preferred embodiment.
Figure 7 shows a cross-section made across the platform, substantially in the plane parallel to the loading platform and across the rotating platform nested within the chassis.

### Detailed Description of the Invention

### Improvements

The present disclosure offers two important improvements over the prior art solutions.

First and most important is the safety when the mobile platform (100) is anchored to the floor. Safety is critical when the medical use is concerned. Most of the time the autonomous mobile platform (100) rests anchored to the floor. As emphasized before, the rest state of the disclosed mobile platform (100) is very similar to an ordinary table with three or more anchoring legs (15), firmly connected to the chassis (20) - or table plane in this analogy. So, sudden hydraulic or electric jack failure cannot destabilize the anchored mobile platform (100), as is possible in cases where the legs (15) are operated by the said jacks, as prior art solutions teach. Furthermore, any accidental drive engagement, executed by program error, will leave the platform in the desired place, eventually spinning the driving wheels at a safe distance above the floor.

The second improvement over the prior art solutions is the possibility for the said autonomous mobile platform (10) to instantaneously move in any desired direction. This is achieved via a specifically designed pair of driving wheels (51, 61), independently operated and attached to the rotating platform (80). The platform (80) is positioned centrally within the said mobile platform (100), where sets of omni-wheels are used for stabilization of the platform (100). The propelling and steering of the platform (100) are performed by the coordinated actions of the rotating plate (80) and separate independent rotation of the left drive (50) and the right drive (60) while the driving wheels (51, 61) and omni-wheels (70) are on the floor.

All these improvements render the disclosed mobile platform (100) better in use when compared with the prior art solutions.

### The platform

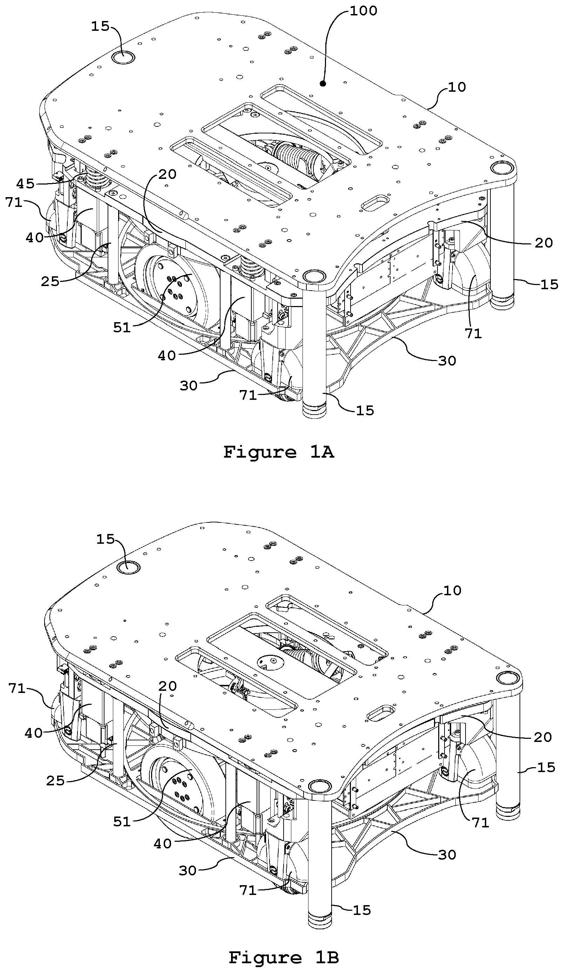

Figures 1A and 1B depict the mobile platform (100) in a state that enables propelling and in an anchored state to the floor, respectively. The autonomous mobile platform (100) for medical use, capable of moving in any direction, comprises a loading platform (10), a chassis (20), a cover (30), two drives (50, 60), a set of omni-wheels (70), a rotating platform (80), and a set of lifting means (40) .

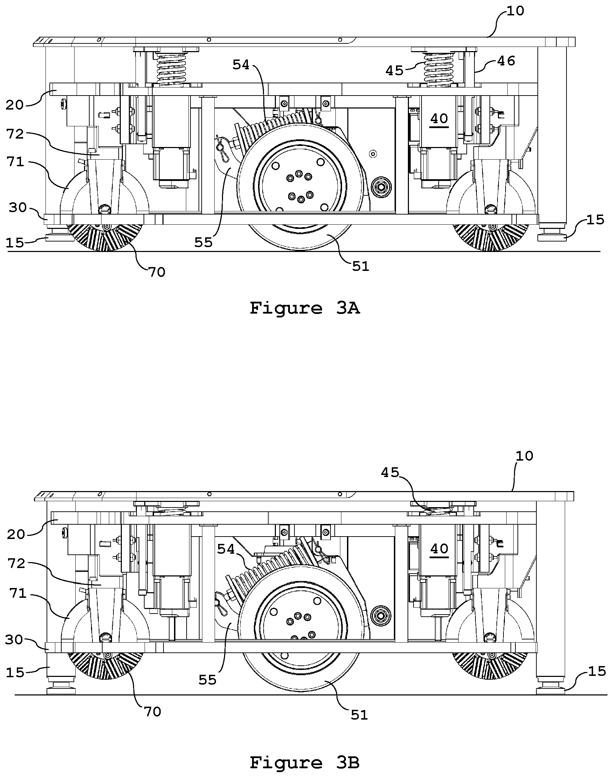

The loading platform (10) is preferably designed as a metal flat plane, with at least three anchoring legs (15) attached to the said plane. The legs (15) number can be arbitrary, however, solutions with three legs have some advantages regarding stability by compensating the floor's imperfections. This loading platform (10) and the firmly fixed anchoring legs (15) have to be manufactured to carry the significant total load of the mobile platform (100) and the useful load, preferably in the form of a medical robot or similar. The upper side of the loading platform is therefore designed to receive the load for performing other tasks, preferably medical tasks. The anchoring legs (15), preferably formed as hollow metal cylinders with the friction foot for better contact with the medical floors, are attached perpendicularly to the loading platform (10), as obvious from Figures 1A and 1B. The anchoring legs (15) are designed long enough to allow the platform (100) to rest on them when the driving means attached to the chassis (20) are retracted towards the said loading platform (10), as visible in Figures 3A and 3B.

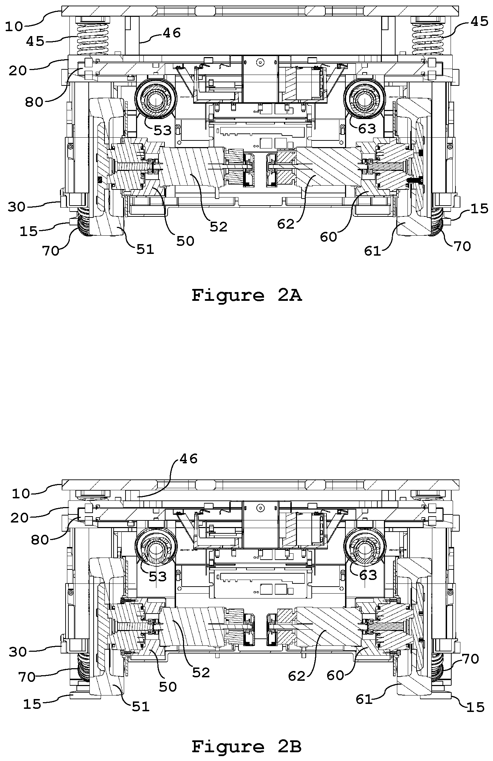

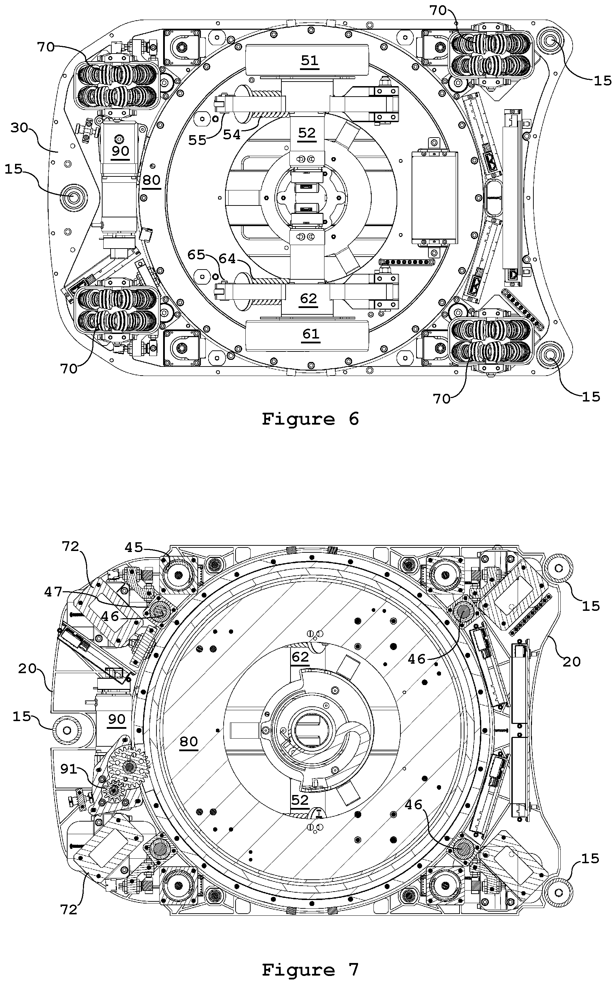

The loading platform (10) is situated above the chassis (20). The chassis (20) is preferably designed as the metal construction capable of carrying all necessary operating means of the mobile platform (100) . The loading platform (10) is stabilized within the said chassis (20) via a set of guides (47), as depicted in Figure 7, which are connected to the chassis (20). The compatible guide rods (46) are connected to the loading platform (10), as depicted in Figures 2A, 3A, and 7, and cooperate with the said guides (47). The guides (47) and compatible guide rods (46) allow the relative motion of the chassis (20) towards the loading platform (10) in a way to keeps the said platforms (10, 20) always parallel.

Lifting and lowering the chassis (20) against the loading platform is performed by using several lifting means (40), depicted in Figures 3A-5B. In the preferred embodiment, four lifting means (40) are used. Each lifting means (40) is fixed to the chassis (20) on one side, and the loading platform (10) on the opposite side. Each lifting means comprises an electromotor (41) which powers a spindle (42) connected to the loading platform (10) and enables lifting and lowering of the chassis (20) towards the loading platform (10). The person skilled in the art may immediately conceive other possibilities, i.e., hydraulic power for the said lifting means (40). However, the electric-driven spindle (42) has advantages in case of electric or mechanical failure or other malfunctions, by keeping the already reached position or level. Also, such construction eliminates the risk of hydraulic fluid leakage which is a critical issue in sterile environments of operating theatres. In the preferred embodiment, four lifting means (40) are used in a coordinated manner to move the chassis (20) relative to the loading platform (10). The spindle (42) geometry and the guides (46) define the maximum distance the chassis (20) can be separated from the loading platform (10).

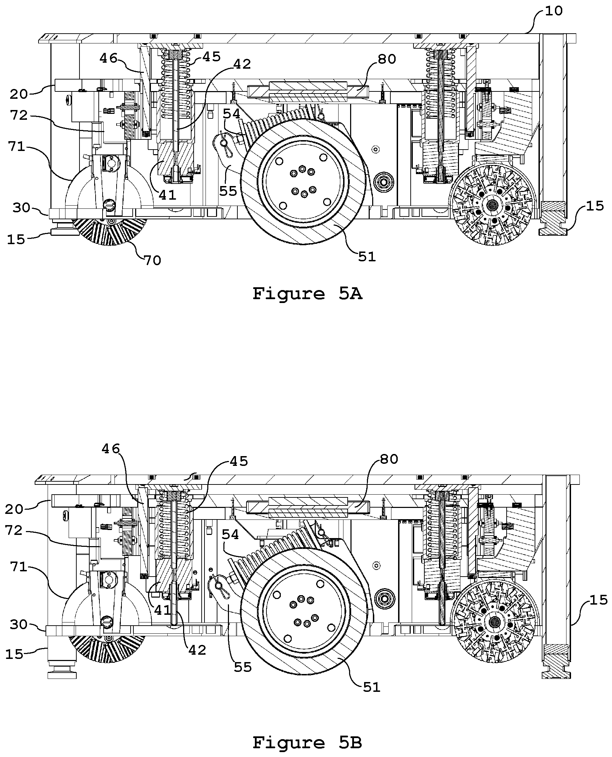

In the preferred embodiment, each lifting means (40) is equipped with a lifting spring (45) situated over the spindle (42), Figures 5A and 5B, to assist with a load. When the chassis (20) is retracted with the lifting means (40) towards the loading platform (10) the retraction stores the potential energy in each spring (45), as depicted in Figure 5B. When the mobile platform (100) is released from the anchoring situation to the position when the platform (100) is ready to move, lowering of the chassis (20) and transfer, i.e., transient of the total load from the legs (15) to the driving wheels (51, 61) and the omni-wheels (70) is assisted by the energy stored in the lifting springs (45). So, such construction enables energy storage to balance the load on lifting spindles (42), i.e., the load is split in both directions rather than the full load being engaged during the lifting session only.

The cover (30) is designed to cover all mechanisms situated below the chassis (20). Preferably, it is manufactured in the form of a metallic grill capable of interconnecting the said mechanisms, i.e., the omni-wheels (70) where necessary. The cover (30) and the chassis (20) are connected via struts (25), preferably four struts (25), as depicted in Figure 1A.

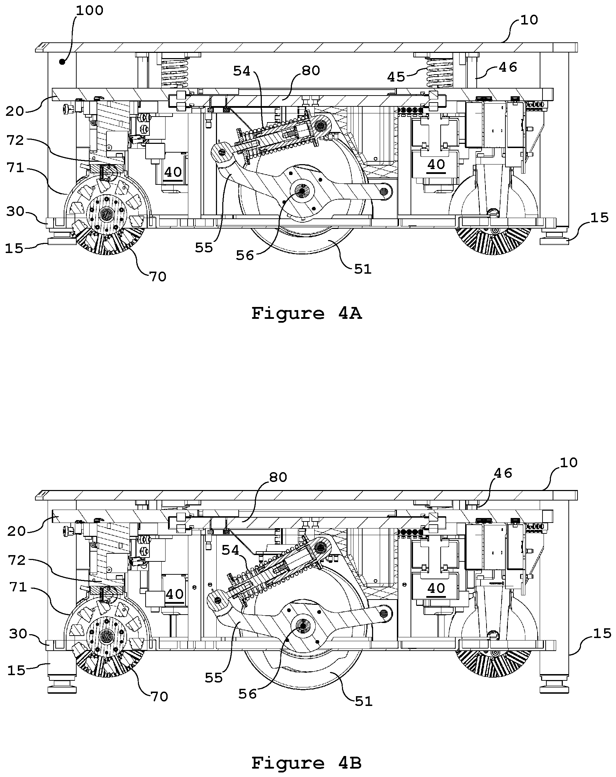

The propulsion system of the mobile platform (100) is formed by the drives (50, 60), each optionally equipped with the electromagnetic brake which is automatically engaged upon the platform (100) stops. Each drive (50, 60) that propels the platform (100) has a driving wheel (51, 61) with a suitable elastomer or tire used for contact with the floor, which is powered by an electromotor (52, 62). This setup is attached to the rotating platform (80) by suspension means (53, 63), depicted in Figures 2A and 2B. In the preferred embodiment, the suspension lever (55, 65) is pivotally fixed to the drive mechanism (50, 60) on one of its sides, the right part in Figures 4A and 4B. At its central part, the suspension lever (55, 65) encloses the bearing with the driving shaft (56, 66). On the opposite end, the suspension lever (55, 65) is pivotally connected to the suspension spring (54, 64) which is again attached to the driving mechanism (50, 60), as depicted in Figures 3A-4B. Finally, the driving means (50, 60) are attached to the rotating platform (80) which is nested within the chassis (20) and rotated by the plate drive (90), as depicted in Figure 7.

To achieve the ability to move from the rest in any desirable direction, the movable platform (100) is equipped with sets of omni-wheels. The omni-wheels are used for the purpose cited in the prior art, i.e., to stabilize the platform (100). The omni-wheels (70) and the drives (50, 60) stabilize the mobile platform (100), while resting on the wheels (50, 60, 70). In the preferred embodiment, each omni-wheel (70) is fixed with the chassis (20) via wheel support (72) and enclosed in wheel casing (71), attached to the cover (30), as depicted in Figure 4A. The omnidirectional wheels or omni-wheels (70) are, preferably, equipped with the elastomers to additionally suspend the platform (100) weight while moving, absorbing the floor imperfections, and acting as the additional suspending means.

The distance between the cover (30) and the chassis (20) is fixed, and smaller than the anchoring legs (15) length, to allow the smooth retraction of the driving mechanism, into the platform (100). The driving means (50, 60), once connected to the rotating platform (80) nested within the chassis (20) is designed so that only part of the driving wheels (51, 61) is situated below the cover (30), as depicted in Figures 3A and 3B, to minimize the opening size. For the person skilled in the art, caring about the end positions of the suspension, achieved with the mechanical limits coupled with the suspension spring (54, 64), is a must to be sure that the driving wheels (51, 61) are above the floor when the driving means (50, 60) are fully retracted. A comparison of Figures 3A and 3B illustrates this problem, the bottom of omni-wheels (70) are positioned differently from the bottom of driving wheels (51, 61), relative to the chassis (20).

Once all this is absolved, the synchronous engagement of the lifting means (40) in one direction lifts the chassis (20) towards the loading platform (10) and leaves the autonomous mobile platform (100) anchored on its anchoring legs (15) on the floor. The driving wheels (50, 60) and all omni-wheels (70) are without any contact with the floor, Figure 3A. The synchronous engagement of the lifting means (40) in the opposite direction moves the chassis (20) from the loading platform (10) and leaves the autonomous mobile platform (100) to stand on the driving wheels (50, 60) and all omni-wheels (70) with all anchoring legs (15) lifted from the floor.

### The kinematics

Few words should be devoted to the autonomous mobile platform (100) kinematics. The described propulsion design is visible in Figure 6. The propelling and steering of the platform (100) are performed by the coordinated actions of the rotating plate (80) and separate independent rotation of the first (left) drive (50) and the second (right) drive (60).

Omni-wheels (70), by definition, can "go" in any direction from the start. However, the mass of the platform (100) may introduce kinematic problems, like driving wheels (51, 61) slippage and the omni-wheels (70) slippage as well. This slippage is greatly reduced by rotating the driving wheels (51, 61) into the desired propagation direction, via a rotating plate (80).

However, a certain correction between the power exerted on the driving wheels (51, 61) may be needed. For that reason, each drive (50, 60) has its electromotor (52, 62) and the on-board computer system corrects the power exerted via electromotors (52, 61) to compensate for the slippage. The rotating plate (80) position helps the platform, (100) to quickly achieve propagation along the desired trajectory, checked via inner or outer telemetry and odometry.

### Industrial Applicability

The present disclosure relates to an autonomous mobile platform (100), comprising a loading platform (10), an upper chassis (20), a lower chassis (30), two centrally positioned independently powered drive wheels (51, 61) with suspension means, a set of omni-wheels (70), a rotating plate (80) and a set of lifting means (40). The synchronous engagement of all lifting means (40) in one direction lifts the connected chassis (20, 30) towards the loading platform (10) and leaves the autonomous mobile platform (100) anchored on their anchoring legs (15) on the floor. The opposite action brings the platform wheels to the floor. The propelling and steering of the platform (100) are performed by the coordinated actions of the rotating plate (80), together with a separate independent rotation of the drive wheels (51, 61), and free rotation of the omni-wheels (70) on the floor. The platform is designed for carrying surgical equipment, especially surgical robots. Therefore, the industrial applicability of the present invention is obvious.

### Reference numbers

- 10: loading platform
- 15: anchoring leg
- 20: chassis
- 25: strut
- 30: cover
- 40: lifting means
- 41: electromotor
- 42: spindle
- 45: lifting spring
- 46: guide rod
- 47: guide
- 50: first (left) drive
- 51: driving wheel
- 52: electromotor
- 53: first drive suspension means
- 54: suspension spring
- 55: suspension lever
- 56: shaft
- 60: second (right) drive
- 61: driving wheel
- 62: electromotor
- 63: second drive suspension means
- 64: suspension spring
- 65: suspension lever
- 66: shaft
- 70: omnidirectional wheel, i.e., omni-wheel
- 71: wheel casing
- 72: wheel support
- 80: rotating plate
- 90: plate drive
- 91: plate gears
- 100: autonomous mobile platform

## Claims

1. An autonomous mobile platform (100) for medical use, capable of moving in any direction, which comprises a loading platform (10), a chassis (20), a cover (30), two drives (50, 60), a set of omni-wheels (70), a rotating platform (80) and a set of lifting means (40), where:
- each drive (50, 60) which propels the platform (100) has a driving wheel (51, 61), powered by an electromotor (52, 62), and is attached to the rotating platform (80) by a suspension means (53, 63),
- the rotating platform (80) is nested within the chassis (20) and rotated by the plate drive (90),
- each lifting means (40) is fixed to the chassis (20) and comprises an electromotor (41) which powers a spindle (42) connected to the loading platform (10) and enables lifting and lowering of the chassis (20) towards the loading platform (10),
- each omni-wheel (70) is fixed with the chassis (20) via wheel support (72) and enclosed in a wheel casing (71),
- the cover (30) is situated below the chassis (20) and their distance is fixed, and
- where the loading platform (10), situated above the chassis (20), is stabilized within the said chassis (20) via a set of guides (47) and compatible guide rods (46), allowing the motion of the chassis (20) towards the loading platform (10) in a way to keeps the said platforms parallel, and where the loading platform (10) is further equipped with at least three anchoring legs (15) of the same height that is longer than the distance between the cover (30) and the chassis (20),
**characterized by that,**
- the synchronous engagement of the lifting means (40) in one direction lifts the chassis (20) towards the loading platform (10) and leaves the autonomous mobile platform (100) anchored on its anchoring legs (15) on the floor, while the driving wheels (50, 60) and all omni-wheels (70) are without any contact with the floor, and
- the synchronous engagement of the lifting means (40) in the opposite direction moves the chassis (20) from the loading platform (10) and leaves the autonomous mobile platform (100) to stand on the driving wheels (50, 60) and all omni-wheels (70) with all anchoring legs (15) lifted from the floor.

2. The autonomous mobile platform (100) according to claim 1, where each lifting means (40) is equipped with a lifting spring (45) situated over the spindle (42) to assist with a load when the chassis (20) is moved, with the lifting means (40), towards the loading platform (10) resulting in the mobile platform (100) on its wheels (70, 51, 61).

3. The autonomous mobile platform (100) according to any of the preceding claims, wherein the propelling and steering of the platform (100) are performed by the coordinated actions of the rotating plate (80) and separate independent rotation of the left drive (50) and the right drive (60) while the driving wheels (51, 61) and omni-wheels (70) are on the floor.

4. The autonomous mobile platform (100) according to claim 3, wherein the left drive (50) and the right drive (60) are equipped with the suspension means (53, 63) formed from the suspension springs (54, 64) pivotally connected to the suspension levers (55, 65) holding the corresponding drive shafts (56, 66) suspended to the rotating plate (80).

5. The autonomous mobile platform (100) according to any of the preceding claims, where the omni-wheels (70) are made of elastomers that act as a suspension means for the said omni-wheels.

6. The autonomous mobile platform (100) according to any of the preceding claims, where the number of the omni-wheels (70) is four and the number of the anchoring legs (15) is three.

7. Use of the autonomous mobile platform (100) for medical use, according to any preceding claims, for carrying surgical equipment, especially surgical robots.
